# EUROPEAN PATENT APPLICATION

(11) **EP 2 229 823 A1**
(43) Date of publication of application: **22.09.2010**
(21) Application number: 09155000.4
(22) Date of filing: 12.03.2009
(51) Int. Cl.: A23L 1/03, A23L 1/30, A23L 1/305, A23J 1/20

(54) **Electrostatic protein/glucosinolate complexes**

(71) Applicant: Nestec S.A., 1800 Vevey (CH)
(72) Inventor: Rade-Kukic, Koraljka, 1018 LAUSANNE (CH); Schmitt, Christophe, 1077, SERVION (CH)
(74) Representative: Marquardt, Ulf

(57) **Abstract**

The present invention relates to the field of electrostatic complexes. Embodiments of the present invention relate to food-grade electrostatic complexes containing at least one milk protein and at least one glucosinolate and to the uses of such complexes, e.g., to reduce the perceived bitterness of glucosinolates and/or to form and stabilize emulsions and/or foams.

## Description

The present invention relates to the field of electrostatic complexes. Embodiments of the present invention relate to food-grade electrostatic complexes containing at least one milk protein and at least one glucosinolate and to the uses of such complexes, e.g., to reduce the perceived bitterness of glucosinolates and/or to form and stabilize emulsions and/or foams.

Glucosinolates (GLS) are natural sulfur-containing phytochemicals that for example are synthesized in vegetables from the Brassicaceae family such as broccoli, kale, cabbage, cauliflower, Brussels sprouts, turnip or mustard greens.

Consumption of these vegetables has been associated, e.g., with reduced incidence of cancer, particularly of lung, stomach, colon, and rectal cancer. Their protective effect is largely attributed to the presence of GLS that possess a common structure comprising a β-D-thioglucose group, a sulfonated moiety, and a variable side chain derived from methionine, tryptophan, phenylalanine or various branched chain amino acids.

GLS occur naturally often as potassium salts. The presence of the sulfate group gives intact GLS strong acidic properties, irrespective of the side chain. Because of the sulfate group and the thioglucose moiety, intact GLS are always water-soluble.

The chemistry of glucosinolates has been extensively described by van Etten and Tookey (Glucosinolates. In CRC Handbook of naturally occurring food toxicants. M Rechcigl Jr (Ed.), CRC Press: Boca Raton, FL. pp 15-30, 1983).

GLS are chemically stable and are considered to be biologically inactive, but when brought into contact with the plant enzyme myrosinase, or human colonic bacteria, they break down releasing glucose and an unstable aglycon. The latter undergoes spontaneous rearrangement into, depending on hydrolysis conditions, different possible products including nitriles, thiocyanates, epithionitriles or isothiocyanates (ITC). ITC are considered to be amongst the most potent naturally occurring bioactive compounds intervening in the process of cancer development. They can affect the endogenous antioxidant potential, enhance detoxification mechanisms and induce apoptosis in undifferentiated cells.

WO0245527 describes a processed dehydrated Brassica vegetable composition containing glucosinolates and endogenous myrosinase enzyme. The endogenous myrosinase converts the glucosinolates into isothiocyanates when the composition is ingested.

GLS are present at relatively high concentrations in the diet compared to some other phytochemicals. For example, a person consuming 3-4 portions of broccoli per week, which is the level at which a protective effect against colorectal adenomas has been reported, might easily be consuming 300-400 mg of GLS.

WO2004089065 A1 describes a method for providing Brassica plants with high levels of anticarcinogenic glucosinolates.

DE102006004829A1 describes a preparation of glucosinolates from root of order *Capparales* in order to produce a food supplement.

WO9907240 describes a formulated food, especially condiment mustard, including crucifer seed or a glucosinolate-containing extract thereof, said seed being other than mustard seed.

WO2006102236A1 describes a composition useful as food product for cancer prevention, skin or hair care product that is free of proteins and comprises natural glucosinolates extracted from a plant.

WO9920242 describes dietetic and cosmetic compositions containing a pectin and a carotenoid, glucosinolate and/or sulforaphan.

Beside their high potential as bioactive molecules, some GLS and their hydrolysis products influence the flavour of *Brassica* vegetables, but also the taste of the food products in which they are added. While being able to provide numerous benefits for the body as described above GLS are known to have a bitter taste.

For example, Drewnowski and Gomez-Carneros (Bitter taste, phytonutrients, and the consumer: a review. 2000. Am J Clin Nutr, 72, 1424-1435) reported that sinigrin and progoitrin, two glucosinolates occurring in Brassicaceae are bitter tasting.

This bitter taste is the reason, why a lot of foods containing glucosinolates are less liked. Because these foods are disliked, only insufficient amounts of glucosinolates are consumed, so that these beneficial compounds cannot produce their beneficial effect.

Consequently, there is a need in the art for a formulation that allows administering glucosinolates while the perception of the bitter taste of glucosinolates is reduced.

It was consequently the object of the present invention to improve the state of the art. In particular, it was the object of the present invention to provide the art with a composition comprising glucosinolates that has no bitter taste or that at least exhibits a reduced bitterness than standard state of the art glucosinolate containing compositions.

The present inventors were surprised to see that they could achieve this object by the subject matter of the independent claims. The dependant claims further develop the idea of the present invention.

The subject matter of the present invention allows it to benefit from the potential bioactive properties of GLS but - at the same time - to reduce the bitter taste induced by these compounds in food compositions.

The present inventors provide electrostatic complexes between GLS and bovine milk proteins, e.g., whey proteins that achieve the object of the present invention.

The inventors have also discovered that these complexes exhibit excellent emulsifying and foaming properties. This further enhances the applicability of the complexes in the food industry.

β-Lactoglobulin (BLG) is a major bovine whey protein. In the present invention, the inventors show that a complex formation between BLG, as one example of a milk protein, and GLS enables reducing the undesirable taste of GLS. This allows it to add GLS to many food products, which so far could not be enriched with GLS due to taste reasons. The present invention allows it now to deliver GLS through various food products. In addition, the formation of electrostatic complexes enables modifying the protein's functional properties, allowing their wider and/or improved usage as food ingredients.

Hence, one embodiment of the present invention is a composition comprising an electrostatic complex. The electrostatic complex comprises at least one glucosinolate and at least one milk protein.

By the term "complex" is meant any form of association between the at least one glucosinolate and at least one milk protein.

The complexes of the invention are electrostatically bound complexes. Thus, the protein preferably has an overall net charge opposite to the overall net charge of the glucosinolate. However, electrostatic complexes formed by means of protein charge patches, i.e. in conditions where the protein and the glucosinolate have similar net charge, are also covered by this application.

Electrostatic complexes occur widely in nature. For example chromatine is an electrostatic complex of DNA and histone proteins. Electrostatic complexation is also utilized in a wide field of applications, e.g. in formulation of personal care products, food products, in coating and in purification technologies. The complexation mechanism may involve both, enthalpic and entropic forces. The enthalpic part in the free energy of association is linked to the pairing of the opposite charges, while the entropic contribution to the free energy may result from the release of counterions, as well as from the loss of degrees of freedom of molecules in their bound state.

The term "milk protein" comprises any protein occurring in milk, as well as any milk or milk derived protein fraction.

The milk may be for example bovine milk, sheep milk, goat milk, horse milk, camel milk or soy milk. Bovine milk is preferred.

In an alternative preferred embodiment of the present invention is the milk protein a whey protein, e.g., sweet whey protein or acid whey protein, preferably from bovine origin.

Hence, the milk protein may be bovine milk or a bovine milk derived protein fraction. For example the milk protein may be selected from the group consisting of the protein fraction of skimmed milk, milk protein concentrate, milk protein isolate, micellar casein, caseinate, αₛ₁-casein, αₛ₂-casein, β-casein, κ-casein, the protein fraction of acid or sweet whey, whey protein concentrate, whey protein isolate, α-lactalbumin, β-lactoglobulin, bovine serum albumin, lactoferrin, or combinations thereof.

Glucosinolates have the following general formula:

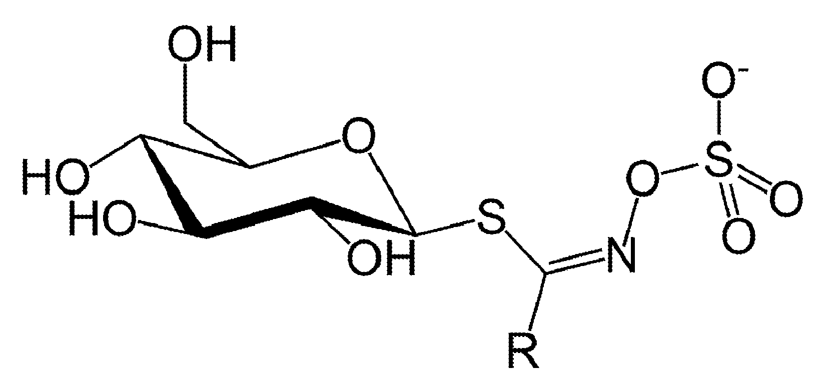

They are a class of organic compounds that contain sulfur and nitrogen and are derived from glucose and an amino acid. The R-group varies.

For example, R may be selected from the group consisting of 2-Propenyl, 4-Hydroxybenzyl, p-Hydroxybenzyl, Benzyl, 2-Phenethyl, 2-Phenylethyl, 3-Butenyl, 4-Methylsulfinylbutyl, 3-Indolylmethyl, 1-Methylpropyl, 4-Pentenyl, 2-Hydroxy-3-butenyl (2R), 2-Hydroxy-3-butenyl (2S), 2-Hydroxy-4-Pentenyl, 3-Methylsulfinylpropyl,3-Methylthiopropyl, 3-Methylsulfonylpropyl, 1-Methoxy-3-indolylmethyl, Methyl, Ethyl, Isopropyl, 2-Propyl, 2-Methylbutyl,(S)2-Hydroxy-2-phenylethyl, p-Methoxybenzyl, m-Methoxybenzyl, 4-Methylthiobutyl, 4-Methylthiobut-3-enyl (Me-S-CH=CH-CH₂-CH₂), 5-Methylthiopentyl, 6-Methylthiohexyl, 8-Methylthiooctyl, 9-Methylthiononyl, 4-(Methylsulfonyl)Butyl, N-acetyl-3-indolylmethyl, and 1-S-6-Methylsulfinyl.

Glucosinolates occur in Brassicalesn including the families Brassicaceae, Capparidaceae and Caricaceae, but also in the genus Drypetes (family Euphorbiaceae). Typical plants that contain glucosinolates include mustard, radish, horseradish, maca, cress, cabbage, Brussels sprouts, kohlrabi, kale, cauliflower, broccoli, turnip, swede and rapeseed.

These plants or glucosinolate containing extracts thereof may be used to produce the complexes of the present invention.

About 120 different glucosinolates are known to occur naturally in plants. They are synthesized from certain amino acids: Aliphatic glucosinolates are derived from methionine, alanine, leucine, valine, or chain elongated forms thereof. Aromatic glucosinolates include Indolic glucosinolates such as glucobrassicin derived from tryptophan and other ones from phenylalanine, its chain elongated homologue homophenylalanine, and sinalbin derived from tyrosine.

In one embodiment of the present invention, the glucosinolate may be selected from the group consisting of aromatic glucosinolates, such as glucosinolates derived from phenylalanine, heterocyclic glucosinolates, e.g., indolyl glucosinolates, such as glucosinolates derived from tryptophan, and aliphatic glucosinolates, such as glucosinolates derived from methionine, alanine, leucine, or valine, or chain elongated homologues of these amino acids, and combinations thereof.

For example, the glucosinolate may be selected from the group consisting of Sinigrin, Sinalbin, Glucosinalbin, Glucotropaeolin (GTL), Gluconasturtiin (GNAST, GST), Gluconapin (GNA), Glucoraphenin, Glucoraphanin, Glucobrassicin, Glucocochlearin, Glucobrassicanapin (GBN), Progoitrin, Epiprogoitrin, Gluconapoleiferin, Glucoiberin, Glucoibeverin, Glucoiberverin (GIV), Glucocheirolin, Neoglucobrassicin (NGBS), Glucoapparin, Glucolepidin, Glucopurtanjivin, Glucoputranjivin, Glucojiaputin, Glucobarbarin (BAR), Glucoaubrietin, Glucolimnanthin, Glucoerucin (GER), Glucoraphasatin, Glucoberteroin, Glucolesquerellin, Glucojirsutin, Glucoarabin, Glucoerysolin, and Glucoalyssin.

The ratio in which the glucosinolate and the milk protein are present in the complex will typically depend on the charge distribution between glucosinolate and milk protein. In may also be influenced by the pH of the medium the complexes are present in.

Typically, the glucosinolate and the milk protein are present in the electrostatic complex in a mole ratio in the range of about 20:1 to 1:20, preferably in a mole ratio in the range of about 10:1 to 1:1, most preferable in the range of about 5:1 to 1:1.

The composition comprising the complexes of the present invention may have any pH, as long as the particular pH allows the formation of the complex of the present invention, in other words as long as at least one milk protein and at least one glucosinolate is oppositely charged. The determination of such a pH-range is within the skill of those skilled in the art.

Typically, however, the composition of the present invention has a pH in the range of about 2 to 9, preferably of about 3 to 7.

It was found that the complexes of the present invention may be used to stabilize emulsions.

An emulsion is a mixture of two immiscible liquids. One liquid (the dispersed phase) is dispersed in the other (the continuous phase). For example an emulsion may be an oil-in-water or a water-in-oil emulsion. Many emulsions are oil/water emulsions, with dietary fats being one common type of oil encountered in everyday life. Examples of emulsions include butter, margarine, milk, cream, mayonnaises and/or vinaigrettes. In milk and cream, water surrounds droplets of fat (an oil-in-water emulsion).

It was shown that the complexes of the invention provide a very good emulsifying stability. Consequently, the complexes can be used to stabilize emulsions over time. Additionally, the complexes of the present invention may also be used to stabilize emulsions under heated conditions.

For example, the present inventors were able to show that the complexes of the present invention allowed it to stabilize an emulsion at an acidic pH (e.g., pH 4) and at elevated temperatures (e.g., 85 °C).

The emulsions remained stable for 26 days without any sign of destabilisation.

Consequently, the complexes of the present invention may be used as emulsifiers and/or stabilisers, for example in food, cosmetic and/or pharmaceutical products.

The composition of the present invention may comprise or consist of an emulsion, preferably an oil-in-water emulsion.

For example, the composition of the present invention may comprise or consist of a foam. A foam is a substance that is formed by dispersing many gas bubbles in a liquid or a semi-solid or in a solid phase.

The composition of the present invention may be a food product, a neutraceutical, a food additive, a medicament, a cream for topical application or a drink.

The compositions of the present invention may also be used for the preparation of a product comprising an emulsion and/or a foam comprising glucosinolates. This product may then be also a food product, a neutraceutical, a food additive, a medicament, a cream for topical application or a drink.

Compositions and/or products comprising the complexes of the present invention are preferably selected from desserts, frozen desserts, dairy products, petfood, culinary products, clinical nutrition products etc. In particular, they may include sauces, soups, mayonnaises, salad dressings, creams, ice cream, chocolate, mousses, and/or milk.

Typical food products may be also selected from the group consisting of fillings, dips, sauces, mayonnaises, spreads, toppings, dairy-based products, milk and/or cream based foams and/or emulsions, a salad dressings, soups, beverages or oral food supplements.

The complexes or compositions of the invention may also be used in cosmetic products such as creams, foams, mousses, gels, shampoos, emulsions, etc.

Pharmaceutical products to which the complexes of the invention may be added include tablets, capsules, syrups, etc.

When used as emulsifier and/or stabiliser, the complexes are preferably present in the product in an amount of 0.01 to 10 wt%, preferably 0.05 - 2 wt%, most preferably 0.1 - 0.5 wt% of said product. It has indeed been found that the emulsifying and stabilising properties are optimal at low concentrations. The products of the invention thus provide the advantage that they are highly effective emulsifiers and/or stabilisers.

The complexes of the present invention may be prepared simply by mixing appropriate amounts of at least one glucosinolate and at least one milk protein in a liquid, preferably water based medium and by allowing the complex to form. Optionally, the pH may be adjusted appropriately and the mixture may be heated, e.g., to 85°C for 15 min and/or agitated.

The resulting complex has the advantage of being food-grade. A material is food-grade if it consists of compounds that are approved for human or animal consumption.

The complexes are highly efficient as an emulsifier and/or stabiliser in compositions to which it is added. Furthermore, the complex comprises only natural ingredients such that it is more appealing than traditional emulsifiers which consist of chemically modified or synthesised products. Also, the complexes of the invention are versatile in terms of the products they may be included into. For instance, by tuning the choice of protein and glucosinolate, they function as emulsifiers and/or stabilisers over a wide pH range. For instance, they can be used in acidic products having a pH of about 4.5 such as mayonnaise as well as in products having a pH over 6.5 such as milk.

Additionally, the solution after complex formation may be subjected to ultrafiltration. This has the advantage of separating the complexes of the invention from free glucosinolates.

In a preferred embodiment, the solution is then dried by any method known in the art such as spray-drying, freeze-drying or vacuum-drying.

The complexes of the invention can therefore be in the form of a solution, a gel, or a dried powder.

In a further embodiment, the protein-glucosinolate complexes may be dried in the presence of further ingredients. Alternatively, the dried hydrolysed protein-glucosinolate complex may be mixed with other dried ingredients.

The resulting products can be for instance a milk powder or dehydrated soup powder comprising the complexes of the invention.

The present inventors could show that if complexed in accordance with the present invention, the glucosinolates do not exhibit their typical bitter taste or at least exhibit a reduced bitter taste.

Eight subjects scored comparatively the bitterness of glucosinolates in free and in complexed form with a nose-clip to eliminate the possible interactions between smell and taste perceptions. It was shown that the formation of electrostatic complexes between protein and glucosinolate enabled a significant reduction of the perceived bitterness of the glucosinolate.

Consequently, the composition of the present invention may be used to improve the taste of glucosinolate containing products, in particular to mask the bitterness of glucosinolates.

The present invention also extends to the complexes and/or compositions of the present invention for the treatment and/or prevention of the disorders mentioned herein.

Those skilled in the art will understand that they can freely combine all features of the present invention described herein, without departing from the scope of the invention as disclosed. In particular, features described for the uses of the present invention may be applied to the composition and product of the present invention and vice versa.

Further advantages and features of the present invention are apparent from the following Examples and Figures.

Figure 1 shows the binding curves of sinigrin (SIN) to β-lactoglobulin (BLG) (with or without heating) at 25°C for pH 4.0 and 7.1. The fit of the binding curve is plotted as a full line together with the dissociation constant K_{D} and the maximum number of binding sites nₘₐₓ. Vertical bars represent standard deviation.

Figure 2 shows volume stability of foams made with BLG-SIN electrostatic complexes at pH 4.0 with (85°C, 15 min) or without heat treatment for different glucosinolate to protein mixing ratio indicated on the charts.

Figure 3 shows the macroscopic appearance of 10% oil-in-water emulsions made at pH 4.0 with 5 mM sinigrin or with BLG-SIN electrostatic complexes (molar ratio 1:10) with (85°C, 15 min) or without heat treatment after 4 and 26 days of preparation. The emulsions were stored at +4°C.

Figure 4 shows sensory scores related to bitterness evaluation of 0.5 mM SIN alone and in the form of an electrostatic complex with BLG after heat treatment (85°C, 15 min), having SIN/BLG molar ratio of 4.

### Examples:

### Example 1: Determination of binding curve of sinigrin to β-lactoglobulin

β-Lactoglobulin (BLG) powder was dispersed in Millipore^{®} water by stirring at room temperature for 1 hour. The protein powder was purchased from Davisco (Biopure, lot JE 001-3-922). The protein content was 93.5 g/100 g of powder, as determined by Kjeldahl analysis (N x 6.38). The composition in the major whey protein fraction was 89.22% BLG, 6.91% β-lactalbumin, 3.87% bovine serum albumin, as determined by reversed phase HPLC (RP-HPLC); 0.2% fat, 1.5% ash and 4.9% moisture, as specified by the supplier. The mineral composition was as follows: 0.87% Na⁺, <0.004% K⁺, <0.04% Cl⁻, 0.019% Ca²⁺, 0.053% P, 0.002% Mg²⁺, as determined by atomic absorption spectroscopy. Protein solubility at pH 4.6 revealed that 96% of the proteins were in native state.

Concentration of the BLG in the protein solution was 1 mM (1.84% w/w). It was stored overnight at 4°C to allow hydration. The next day, the protein dispersion was filtered using a Stericup filtration system (Millipore^{®}) with 0.22 µm GP Express Plus membrane. The filtered dispersion (pH 7.2) was either used directly for sample preparation, or it was previously acidified to pH 4.0 using 1M HCl. The amount of 1M HCl added in the latter case was about 1% of the total volume.

The glucosinolate solution was prepared on the day of the experiment by dissolving sinigrin (SIN) in Millipore^{®} water to obtain a concentration 20 or 40 mM (0.83. or 1.66 % w/w). Sinigrin hydrate (allyl glucosinolate) 99%, was purchased from Sigma (lot 1291054). For the experiments under acidic conditions, SIN solution was acidified to pH 4.0. The amount of 1M HCl added in the latter case was about 1% of the total volume. The solution was then filtered using a syringe filter with 0.22 µm pore-size PVDF membrane (MILLEX^{®} -GV, Millipore; Milian, Switzerland).

At both neutral (7.2) and acidic (4.0) pH, binding of SIN to BLG was investigated under 2 conditions:
• Without heat treatment: BLG and SIN mixture was incubated 1 hour at room temperature;
• With heat treatment: BLG and SIN mixture was heated at 85°C for 15 minutes and cooled down to room temperature within 40 minutes.

A volume of 20 mL was prepared by mixing 1 mM BLG (pH 7.2 or pH 4.0) with 20 mM SIN (pH 5.3) or 40 mM SIN (pH 4.0) in Millipore^{®} water to obtain a final SIN to BLG molar ratio ranging from 2 to 20 at pH 7.2, and from 2 to 40 at pH 4.0. The pH of solutions after mixing was not further adjusted. Control samples of BLG alone were prepared as well.

### Incubation of the samples was done as follows:

Without heat treatment, 8 mL of each BLG/SIN mixture was incubated at room temperature for 1 hour, and then put on ice until further analysis.

With heat treatment, 12 mL of each BLG/SIN mixture was put into 12 mL-glass vial and heated on 85°C for 15 min, in addition to 5 min necessary for temperature in the vials to reach 85°C. The heating was done without agitation, in a temperature-controlled water-bath. Subsequently, samples were stirred, let to cool down at room temperature over 40 minutes, and then put on ice until further analysis.

After incubation, 2 x 1.5 mL of mixtures were centrifuged using Centrisart^{®} I centrifugal ultrafiltration units with cellulose triacetate ultrafilters having molecular weight cut-off of 10 kDa (Sartorious, Germany) in a Sorvall RC 3C PLUS centrifuge with a swing bucket rotor (Thermo, Switzerland), for 30 min at 2900 x g. Ultrafiltrates were collected, diluted 10 times in Millipore^{®} water, and then analyzed by size exclusion HPLC (SEC) to determine the amount of free (unbound) SIN in each sample.

SEC was performed using an Agilent 1100 Series System HPLC equipped with an autosampler and an UV detector. TSKGEL G2000 SWXL (30cm x 7.8mm ID) column with a corresponding guard-column (TosoHaas Stuttgart, Germany) were used. The eluent was 20 mM phosphate buffer at pH 7.1, containing Na₂HPO₄ and NaH₂PO₄ in Millipore^{®} water, filtered on a Stericup filtration system with 0.22 µm GP Express Plus membrane prior to use. The chromatography was carried out by injecting 50 µL of samples, at 25°C and with the flow of 0.8 mL/min (the time of one run was 20 min). The detection was done at 228 nm (maximal absorption of SIN) and 280 nm (absorption of SIN at this wavelength is negligible). BLG absorbs at both wavelengths. Concentrations of BLG and SIN were assessed according to previously made standard calibration curves.

Binding curves were obtained by plotting molar binding ratio of SIN to BLG (B_{SIN/BLG}) against free (unbound) SIN. The best-fit values for binding parameters (K_{D} and nₘₐₓ) were achieved by applying non-linear least-squares regression using the software Microcal Origin 6.0 (Microcal Software Inc., Northampton, USA). For all the samples equation for one set of binding sites was applied.

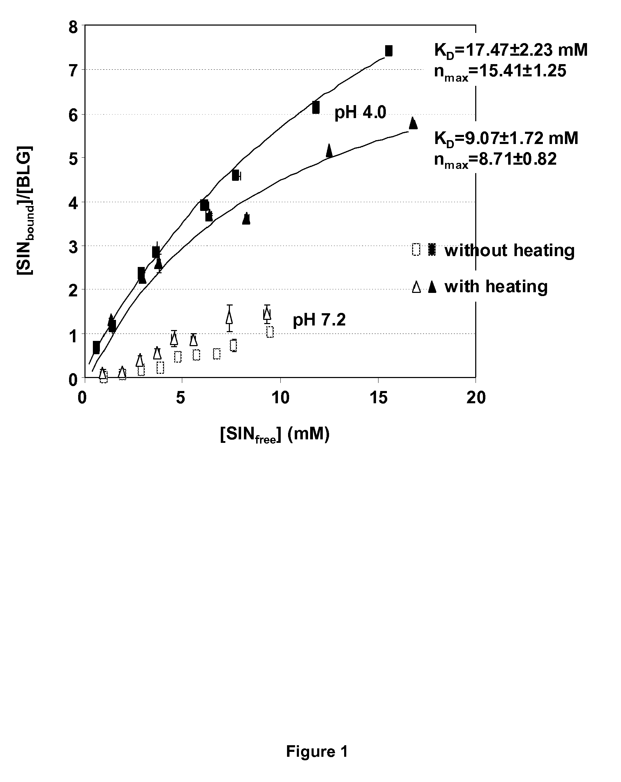

Figure 1 shows that the amount of bound SIN to BLG was much higher at pH 4.0 compared to 7.2. As the iso-electrical pH of BLG is around 5.2, the protein is mainly positively charged at pH 4.0 and negatively charged at pH 7.0. The results indicate the formation of electrostatic complexes between BLG and SIN in acidic conditions. The heat treatment had an effect on the binding of the glucosinolate on the protein, since the number of maximum binding sites decreased from 15.4 to 8.71. This might be related to some protein aggregation induced by heat.

### Example 2: Determination of the foaming properties of β-lactoglobulin/sinigrin electrostatic complexes

Foaming properties of samples (prepared as described in example 1) were evaluated using the standardized method proposed by Guillerme C, Loisel W, Bertrand D, Popineau Y. Study of foam stability by video image analysis: Relationship with the quantity of liquid in the foams. 1993. J Text Stud, 24, 287-302. The apparatus used was a standard version of the Foamscan^{™} from Teclis - ITConcept (Longessaigne, France). The principle of the method is to foam a definite quantity of protein dispersion by infusing gas into it through a glass frit of specific porosity. The gas flow and duration of the bubbling are controlled. The foam is generated on the surface of the liquid and it rises in the glass tube where its height is followed in real time by image analysis using a charged-coupled device (CCD) camera. The amount of liquid incorporated into the foam and the drainage rate are followed by measuring the conductivity as a function of time and with reference to the conductivity of the solution before bubbling, in the cuvette (with the remaining liquid phase), and at electrodes placed at different heights of the tube (Kato A, Takahashi A, Matsudomi N, Kobayashi K. Determination of foaming properties of proteins by conductivity measurements. 1983. J Food Sci, 48, 62-65).

A volume of 20 mL of sample at pH 4 (SIN to BLG molar ratio 4, 10 and 20 with or without heat treatment) was diluted to 55 µM BLG (0.1% w/w) and placed in the cuvette of the Foamscan^{™}. Nitrogen at the flow rate 80 mL/min was bubbled in through the glass frit of porosity 4, creating the air bubbles with diameters between 10 and 16 µm. This flow rate allowed efficient foam formation before strong gravitational drainage occurred. The bubbling was stopped when the foam volume of 110 cm³ was reached. Subsequently, foam capacity (FC = volume of foam/volume of gas injected) and foam expansion (FE = volume of the foam/volume of the liquid in the foam) were calculated. Foam volume and liquid stability (time for the foam to drain 50% of its initial liquid content) were followed over next 30 min at 24+1°C.

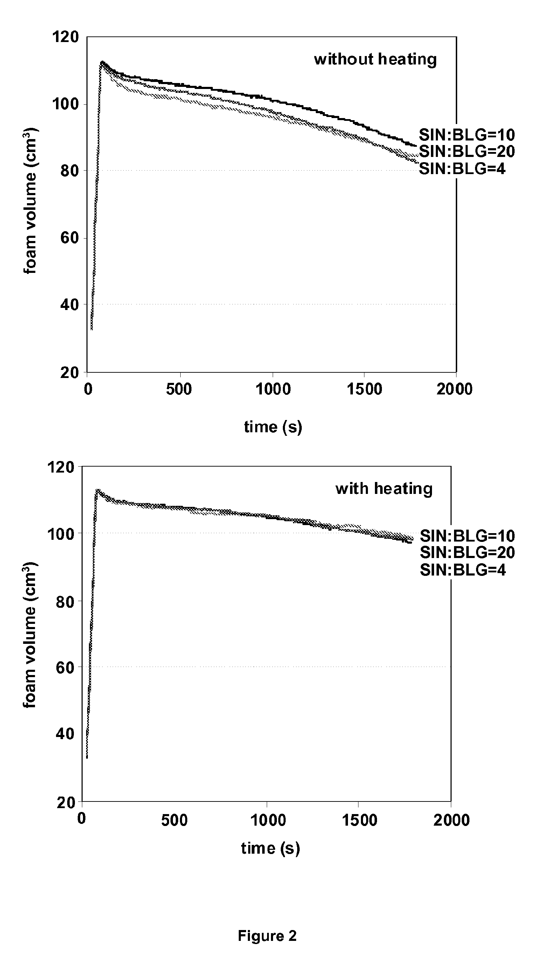

Figure 2 shows that foams produced with electrostatic complexes without heat treatment were volume stable, losing about 20% of their initial volume after 30 minutes, whatever the SIN/BLG molar ratio. This was an indication of the surface activity of the complexes at the air/water interface. Upon heating of the electrostatic complexes, foams became much more volume stable than previously, the volume loss being only 10% on the same timescale.

**Table 1** shows the foam expansion, foam capacity and foam liquid stability determined at pH 4.0 for non-heated and heated (85°C, 15 min) BLG-SIN electrostatic complexes at 25°C for SIN/BLG molar mixing ratio of 4, 10 and 20. Reported data are mean values with corresponding standard deviation.

| | | **foam expansion** | **foam capacity** | **foam liquid stability (s)** |
|---|---|---|---|---|
| **without heating** | SIN:BLG | 9.0±0.2 | 1.14±0.01 | 131±4.2 |
| | ratio 4 | | | |
| | SIN:BLG | 9.1±0.1 | 1.12±0.01 | 137.5±5.5 |
| | ratio 10 | | | |
| | SIN:BLG | 8.9±0.2 | 1.13±0.01 | 135.0±4.0 |
| | ratio 20 | | | |
| **with heating** | SIN:BLG | 9.9±0.2 | 1.14±0.01 | 147.7±1.9 |
| | ratio 4 | | | |
| | SIN:BLG | 9.9±0.2 | 1.14±0.02 | 149.0±1.0 |
| | ratio 10 | | | |
| | SIN:BLG | 10.0±0.5 | 1.13±0.03 | 144.5±3.5 |
| | ratio 20 | | | |

The table 1 reports on the foaming and foam stabilizing properties of the BLG-SIN electrostatic complexes. No significant effect of molar ratio or heat treatment was observed on the foam expansion. Similarly, the foam capacity of the electrostatic complexes was not affected by the molar ratio or the heat treatment. The foam liquid stability was however slightly improved for the heated electrostatic complexes.

### Example 3: Determination of the emulsifying properties of β-lactoglobulin/sinigrin electrostatic complexes

Emulsifying properties of samples at pH 4.0 (prepared as described in example 1) were evaluated by preparing emulsions containing 10% (w/w) of "high oleic" sunflower oil (Oleifico SABO, Manno, Switzerland). Samples with SIN to BLG molar ratio 10 with or without heat treatment were diluted to 0.25 mM BLG and mixed with oil in a Pyrex^{®} tube (D 25 mm, V 35 mL) to a final weight of 20 g. They were pre-homogenized using an Ultra Turrax^{®} T25 equipped with a S25N-10G dispersing head (IKA-Werke, Staufen, Germany) rotating at 16,000 rpm for 1 minute. Homogenization of emulsions was completed using an ultrasonic processor (Hielscher UP400S, power 400 W, frequency 24 kHz) and a sonotrode (diameter 7 mm, approx. length 100 mm, titanium) at room temperature for 75 seconds and 75% amplitude of the ultrasound maximal power. This emulsification time was enough to disperse the oil in small droplets and to avoid excessive heating of the sample. The temperature inside the samples did not surpass 50°C.

Stability of emulsions to creaming and flocculation was followed over 26 days of storage at 4°C.

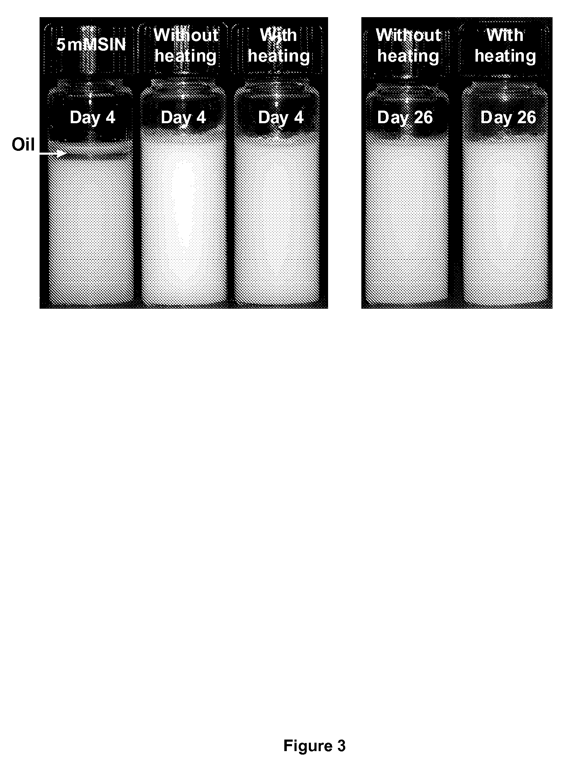

Figure 3 shows that SIN alone was not able to produce a stable oil/water emulsion, as oiling-off was already visible after emulsification. Use of SIN-BLG electrostatic complexes enabled to produce very stable emulsion at pH 4.0, without or with heat treatment applied to the complexes. This result indicates that the complexes exhibited interfacial activity at the oil/water interface. After 26 days of storage at 4°C, there was no macroscopic evidence of creaming or flocculation in the emulsions stabilized by the electrostatic complexes, indicating the stability of the interfacial layer covering the oil droplets.

### Example 4: Determination of the bitterness of β-lactoglobulin/sinigrin electrostatic complexes

Eight subjects were instructed to comparatively score bitterness, on the scale from 0 (not) to 10 (very), of solutions containing 0.5 mM BLG, 2 mM SIN or 0.5 mM BLG+2 mM SIN at pH 4 (prepared as described in example 1, with heat treatment). Samples were presented in a randomized order in 2cl cups and coded. Subjects were instructed to first evaluate samples in a precise order (from left to right). Once they had tasted each sample, they were free to taste any sample another time. Vittel^{™} mineral water at ambient temperature, bread or apple slices were available for mouth rinsing. To eliminate the possible interactions between smell and taste perceptions, evaluations were done with the nose-clip. To determine if there was significant difference between samples, pair-wised t-tests was applied.

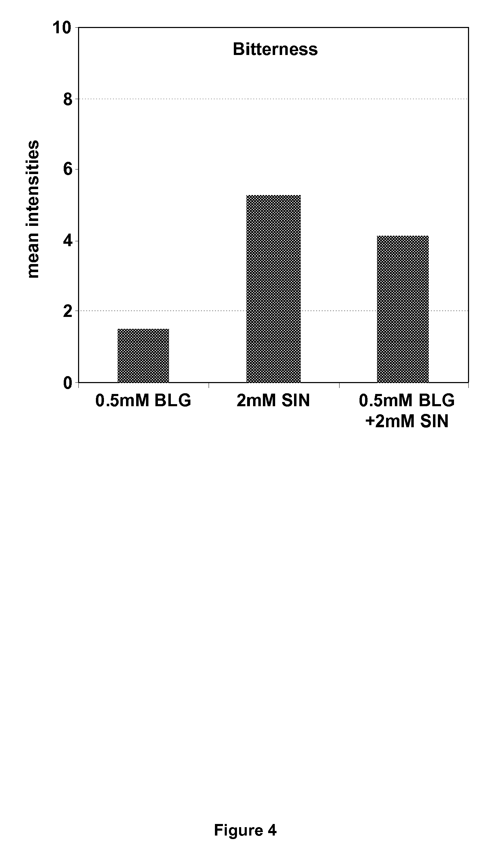

Figure 4 indicates that formation of electrostatic complexes between SIN and BLG enables significant reduction of the perceived bitterness of SIN.

## Claims

1. Composition comprising an electrostatic complex comprising at least one glucosinolate and at least one milk protein.

2. Composition in accordance with claim 1, wherein the milk protein is selected from the group consisting of the protein fraction of bovine milk, the protein fraction of skim milk, milk protein concentrate, milk protein isolate, micellar casein, caseinate, αₛ₁-casein, αₛ₂-casein, β-casein, K-casein, the protein fraction of acid or sweet whey, whey protein concentrate, whey protein isolate, α-lactalbumin, β-lactoglobulin, bovine serum albumin, lactoferrin, or combinations thereof.

3. Composition in accordance with one of the preceding claims, wherein the glucosinolate is selected from the group consisting of aromatic glucosinolates, such as glucosinolates derived from phenylalanine, heterocyclic glucosinolates, e.g., indolyl glucosinolates, such as glucosinolates derived from tryptophan, and aliphatic glucosinolates, such as glucosinolates derived from methionine, alanine, leucine, or valine, or chain elongated homologues of these amino acids, and combinations thereof.

4. Composition in accordance with one of the preceding claims, wherein glucosinolate and milk protein are present in the complex in a mole ratio in the range of about 20:1 to 1:20, preferably in a mole ratio in the range of about 10:1 to 1:1, most preferable in the range of about 5:1 to 1:1.

5. Composition in accordance with one of the preceding claims, wherein the composition has a pH in the range of about 2 to 9, preferably of about 3 to 7.

6. Composition in accordance with one of the preceding claims, wherein the composition comprises, preferably consists of, an emulsion, preferably an oil-in-water emulsion.

7. Composition in accordance with one of the preceding claims, wherein the composition comprises, preferably consists of, a foam.

8. Use of a composition in accordance with one of claims 1-9 for the production of a product comprising an emulsion and/or a foam comprising glucosinolates.

9. Use in accordance with claim 8, wherein the product is a food product, a neutraceutical, a food additive, a medicament, a cream for topical application or a drink.

10. Use of a composition in accordance with one of claims 1-9 to improve the taste of a glucosinolate containing product, in particular to mask the bitterness of glucosinolates.

11. Use in accordance with claim 10, wherein the product is selected from the group consisting of fillings, dips, sauces, mayonnaises, spreads, toppings, dairy-based products, milk and/or cream based foams and/or emulsions, a salad dressings, soups, beverages or oral food supplements.
